(19) **Europäisches Patentamt / European Patent Office / Office européen des brevets**

(11) **EP 3 255 411 A1**

(12) # EUROPEAN PATENT APPLICATION
published in accordance with Art. 153(4) EPC

(43) Date of publication:
**13.12.2017 Bulletin 2017/50**

(21) Application number: **15881155.4**

(22) Date of filing: **24.09.2015**

(51) Int Cl.:
***G01N 21/3504*** (2014.01)

(86) International application number:
**PCT/JP2015/076995**

(87) International publication number:
**WO 2016/125338 (11.08.2016 Gazette 2016/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **06.02.2015 JP 2015022683**

(71) Applicant: **Kabushiki Kaisha Toshiba
Minato-ku
Tokyo 105-8001 (JP)**

(72) Inventors:
• **TAKAGI, Shigeyuki
Tokyo 105-8001 (JP)**
• **KAKUNO, Tsutomu
Tokyo 105-8001 (JP)**
• **SHIOMI, Yasutomo
Tokyo 105-8001 (JP)**
• **MAEKAWA, Akira
Tokyo 105-8001 (JP)**
• **KUSABA,Miyuki
Tokyo 105-8001 (JP)**
• **HASEGAWA, Hiroshi
Tokyo 105-8001 (JP)**
• **MAGARA, Takashi
Tokyo 105-8001 (JP)**

(74) Representative: **Hoffmann Eitle
Patent- und Rechtsanwälte PartmbB
Arabellastraße 30
81925 München (DE)**

(54) **GAS ANALYZING METHOD AND GAS ANALYZING DEVICE**

(57) A gas analysis method includes irradiating a sample gas introduced into a gas cell with infrared light tuned to a wavelength corresponding to one absorption line of a target gas contained in the sample gas, measuring a sample signal value corresponding to intensity of transmitted light of the infrared light transmitted through the gas cell, evacuating the sample gas in the gas cell and then replacing by a reference gas, measuring a reference signal value corresponding to intensity of transmitted light of the infrared light transmitted through the reference gas, and calculating gas concentration at the one absorption line from ratio of the sample signal value to the reference signal value.

FIG. 2

**Description**

[Technical Field]

This invention relates to a gas analysis method and a gas analysis device.

[Background Art]

The laser device emitting infrared light is applied to a gas analysis device.

Most gases absorb infrared radiation in accordance with their intrinsic absorption spectra. The absorbance depends on gas concentration. The absorbance can be determined by measuring transmitted light intensity with respect to the incident light intensity of infrared light. Thus, the concentration of a gas can be determined by measuring the transmission intensity of infrared light in the gas.

For instance, when the gas is expired breath, the amount of e.g. $CO_2$, $NO_2$, $NH_3$, acetone, and methane contained therein is very small. Thus, it is required to improve the accuracy of gas concentration measurement.

[Prior Art Document]

[Patent Document]

[Patent Document 1] JP 4108297 B2

[Summary of Invention]

[Problem to be Solved by Invention]

An object of the invention is to provide a gas analysis method and a gas analysis device that can measure gas concentration with high accuracy.

[Means for Solving Problem]

According to one embodiment, a gas analysis method includes irradiating a sample gas introduced into a gas cell with infrared light tuned to a wavelength corresponding to one absorption line of a target gas contained in the sample gas, measuring a sample signal value corresponding to intensity of transmitted light of the infrared light transmitted through the gas cell, evacuating the sample gas in the gas cell and then replacing by a reference gas, measuring a reference signal value corresponding to intensity of transmitted light of the infrared light transmitted through the reference gas, and calculating gas concentration at the one absorption line from ratio of the sample signal value to the reference signal value.

[Brief Description of Drawings]

[FIG. 1] FIG. 1 is a configuration view of a gas analysis device according to this embodiment.
[FIG. 2] FIG. 2 is a flow chart of a gas analysis method according to a first embodiment.
[FIG. 3] FIG. 3 is a flow chart of a gas analysis method according to a comparative example.
[FIG. 4] FIG. 4A is a graph showing the light intensity waveform in the comparative example. FIG. 4B is a graph showing the light intensity waveform in the first embodiment.
[FIG. 5] FIG. 5A is a graph showing the dependence of reference gas transmitted light intensity on the cumulative number of times in the comparative example. FIG. 5B is a graph showing the dependence of reference gas transmitted light intensity on the cumulative number of times in the first embodiment.
[FIG. 6] FIG. 6 shows a first variation of the gas analysis device of this embodiment.
[FIG. 7] FIG. 7 is a flow chart of a gas analysis method according to a second embodiment.
[FIG. 8] FIG. 8 is a flow chart of a gas analysis method according to a third embodiment.
[FIG. 9] FIG. 9 is a flow chart showing an alternative example of the sequence for outputting an alarm signal.
[FIG. 10] FIG. 10 is a configuration view of measuring the gas concentration off-line.

[Embodiments of Invention]

Embodiments of the invention will now be described with reference to the drawings.

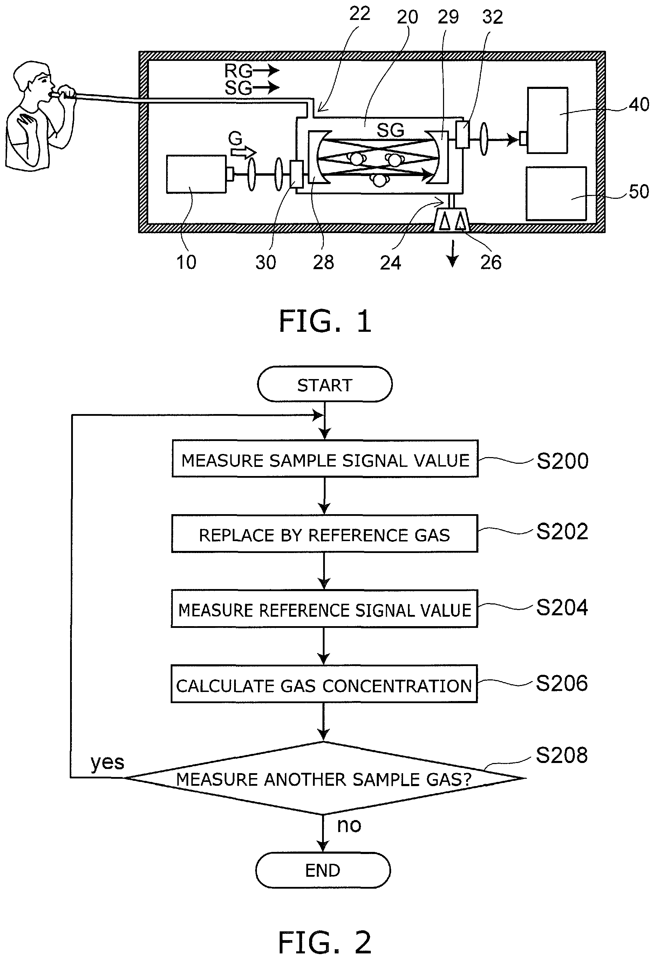

FIG. 1 is a configuration view of a gas analysis device according to this embodiment.

The gas analysis device 5 includes a light source 10, a gas cell 20, a detection section 40, and a control section 50.

The light source 10 emits infrared light G. The wavelength of the infrared light G can be tuned to an absorption line of the target gas contained in the sample gas SG. The light source 10 may be e.g. a QCL (quantum cascade laser), semiconductor laser, or VCSEL (vertical cavity surface emitting laser). Then, the infrared light G can be laser light having high optical density. The wavelength of the infrared light G may be set to e.g. 3-12 μm. This enables measurement of the concentration of many organic gases and inorganic gases.

The gas cell 20 has an introduction port 22 and an ejection port 26. A reference gas RG and a sample gas SG are selectively introduced into the gas cell 20. A vacuum pump 26 may be provided outside the ejection port 24. Then, the gas can be switched rapidly.

The gas cell 20 includes an incoming section 30 and an outgoing section 32. The incoming section 30 is irradiated with infrared light corresponding to a first absorption line among the absorption lines of one gas. The infrared light travels out of the outgoing section 32.

The detection section 40 detects a sample signal value corresponding to the transmitted light intensity I of the sample gas SG with respect to the reference signal value corresponding to the transmitted light intensity IO of the reference gas RG. The reference gas RG has sufficiently low optical absorption. The detection section 40 can be e.g. a cooled detector (made of MCT (HgCdTe)) or photodiode.

The control section 50 selects a gas introduction sequence so as to measure the sample signal value earlier than the reference signal value at the first absorption line. The control section 50 calculates the gas concentration c at one absorption line from the ratio of the sample signal value to the reference signal value.

The absorbance A is given by equation (1).

$$A = -\ln\left[\frac{I}{I_O}\right] = -\ln T = \alpha L \tag{1}$$

where A: Absorbance
$I_O$: Incident Light Intensity
I: Transmitted Light Intensity
T (=I/IO): Transmittance
α: Absorption Coefficient
L: Optical Length

The absorption coefficient α is given by equation (2).

$$\alpha = \varepsilon c \tag{2}$$

where c: Gas Concentration
ε: Molar Absorption Coefficient

The gas concentration c can be calculated from equation (3).

$$c = -\left[\frac{1}{\varepsilon L}\right] \times \ln T \tag{3}$$

Concave mirrors 28, 29 opposed to each other can be placed in the gas cell 20. This provides a multipath configuration and can substantially lengthen the optical length L of the infrared light G. Thus, the absorbance A can be improved by equation (1).

FIG. 2 is a flow chart of a gas analysis method according to a first embodiment.

The gas analysis device of the embodiment shown in FIG. 1 can analyze various gases such as environmental

gas, $H_2O$, chemical substances, and biogas. This figure illustrates an example in which the sample gas SG is human expired breath. It is understood that the sample gas SG is not limited to expired breath.

First, the gas analysis device is switched on. In this case, the light source 10 can be switched on.

Next, a sample signal value is measured (S200). Step S200 includes the step of introducing a sample gas SG, tuning the wavelength of infrared light to one of the absorption lines of the sample gas SG, irradiating the sample gas SG with the infrared light, and measuring the transmitted light intensity I. When the target sample gas SG contains a plurality of gas components, the sample signal value is measured at a plurality of absorption lines.

Next, the sample gas SG in the gas cell 20 is replaced by a reference gas RG containing air (S202).

Next, a reference signal vale is measured (S204). Step S204 includes the step of tuning the wavelength of infrared light to one of the absorption lines of the sample gas SG, irradiating the reference gas with the infrared light, and measuring the transmitted light intensity I. When the target sample gas SG contains a plurality of gas components, the reference signal value is measured at a plurality of absorption lines.

The control section 50 calculates the gas concentration c at one absorption line from the ratio of the sample signal value to the reference signal value using e.g. equations (1)-(3) (S206).

When another sample gas SG is measured (yes), the flow returns to step S200. In this case, the gas cell 20 has been filled with the reference gas RG. Thus, the degree of contamination therein is low. This enables introduction of a sample gas SG in step S200. When another sample gas SG is not measured (no), the measurement is ended.

For instance, measuring a trace amount of $CO_2$, $NO_2$, $NH_3$, acetone, and methane contained in human expired breath facilitates prevention and early detection of various diseases.

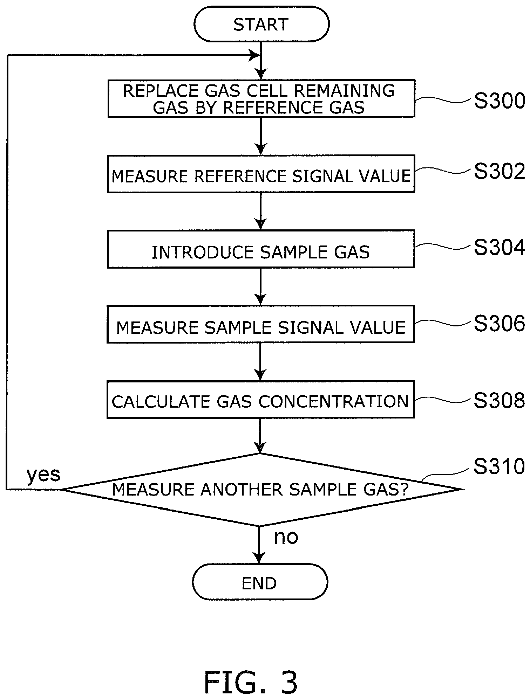

FIG. 3 is a flow chart of a gas analysis method according to a comparative example.

The gas analysis device is the same as that of FIG. 1. However, the gas introduction sequence is different.

First, the gas remaining in the gas cell 20 is ejected. The gas cell 20 is cleaned by introducing e.g. air, and then replaced by a reference gas RG (S300).

Next, a reference signal vale is measured (S302). Step S302 includes the step of tuning the wavelength of infrared light to one of the absorption lines of the sample gas SG, irradiating the reference gas RG with the infrared light, and measuring the transmitted light intensity I0. When the target sample gas SG contains a plurality of gas components, the reference signal value is measured at a plurality of absorption lines.

Next, the gas cell 20 is replaced by a sample gas SG containing air (S304).

Next, a sample signal value is measured (S306). Step S306 includes the step of tuning the wavelength of infrared light to one of the absorption lines of the sample gas SG, irradiating the sample gas SG with the infrared light, and measuring the transmitted light intensity I0. When the target sample gas SG contains a plurality of gas components, the sample signal value is measured at a plurality of absorption lines.

The control section 50 calculates the gas concentration c at a first absorption line from the ratio of the sample signal value to the reference signal value using e.g. equations (1)-(3) (S308).

When another sample gas SG is measured (yes), the flow returns to step 300. In this case, the gas cell 20 is filled with the sample gas for a long time. Thus, the inside of the gas cell 20 is often contaminated. This requires cleaning of step 300. Thus, the measurement time is longer compared with the first gas analysis method. When another sample gas SG is not measured (no), the measurement is ended.

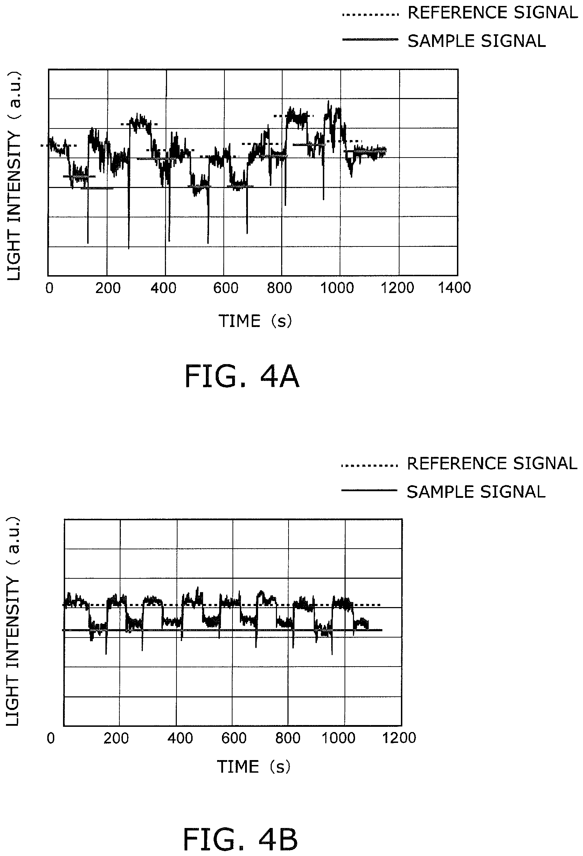

FIG. 4A is a graph showing the light intensity waveform in the comparative example. FIG. 4B is a graph showing the light intensity waveform in the first embodiment.

In the comparative example of FIG. 4A, both the reference signal value indicated by the dashed line and the sample signal value indicated by the solid line have large temporal variation. Thus, the variation of the ratio between the two signal values is large and decreases the measurement accuracy. On the other hand, in the first embodiment shown in FIG. 4B, both the reference signal value indicated by the dashed line and the sample signal value indicated by the solid line have small change and are stable. Thus, the change of the ratio between the two signal values is also reduced. Accordingly, the measurement accuracy can be improved.

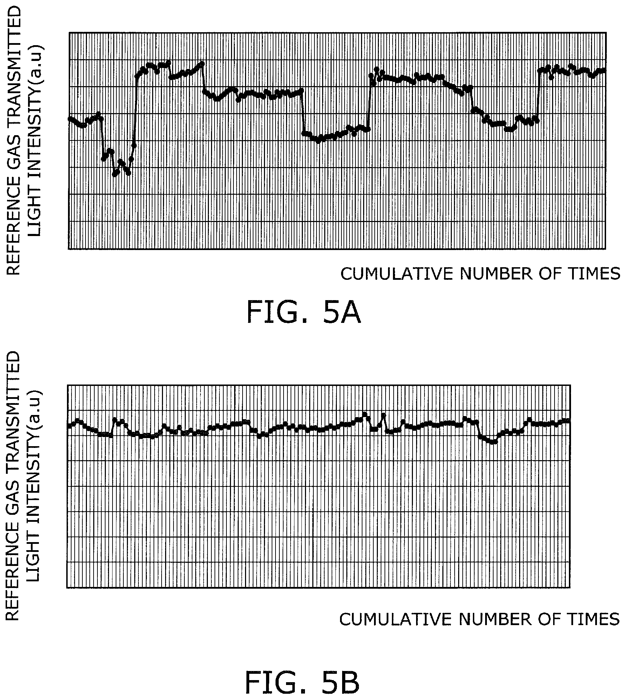

FIG. 5A is a graph showing the dependence of reference gas transmitted light intensity on the cumulative number of times in the comparative example. FIG. 5B is a graph showing the dependence of reference gas transmitted light intensity on the cumulative number of times in the first embodiment.

FIGS. 5A and 5B show the result of measuring the light intensity of light transmitted through the reference gas when the gas analysis device is operated for approximately eight days. In the comparative example of FIG. 5A, the gas cell 20 is filled with the sample gas during the period when the gas analysis device does not perform measurement. Thus, the change of the light intensity of infrared light transmitted through the reference gas is large. The change of light intensity becomes large particularly when no measurement is performed for a long time. On the other hand, in the first embodiment of FIG. 5B, the inside of the gas cell 20 is filled with the reference gas such as air at the end of one measurement cycle. Thus, even in the operation for approximately eight days, contamination inside the gas cell 20 is suppressed, and the change of light intensity is small. This allows high accuracy measurement even if the next measurement cycle is started with the introduction of the sample gas.

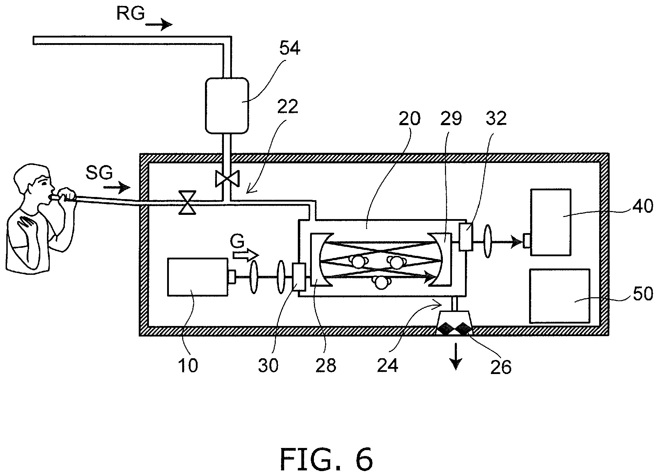

FIG. 6 shows a first variation of the gas analysis device of this embodiment.

The gas analysis device includes a light source 10, a gas cell 20, a detection section 40, a control section 50, and an adsorption column 54.

The adsorption column 54 is provided in the path for introducing the reference gas RG such as air. The adsorption column 54 can be e.g. a filter containing an impurity adsorbent material.

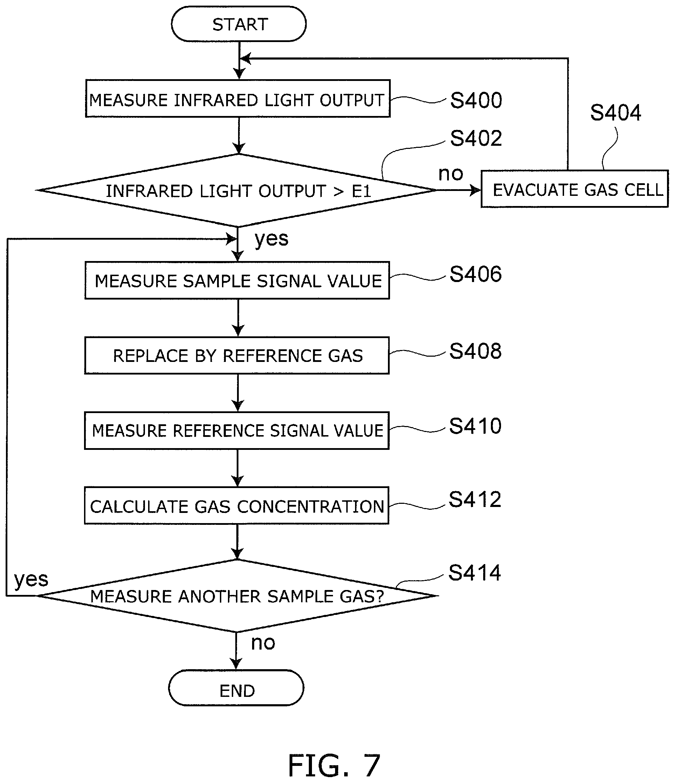

FIG. 7 is a flow chart of a gas analysis method according to a second embodiment.

In the second embodiment, first, it is determined whether the light intensity of infrared light is higher than a prescribed value.

The gas measurement device is switched on. Then, the light intensity of infrared light is measured (S400) before introducing a sample gas SG into the gas cell 20.

Next, when the light intensity of infrared light is less than or equal to the prescribed value E1, the gas cell 20 is evacuated and cleaned (S404). If the transmitted light $I_0$ of infrared light is higher than the prescribed value E1, the degree of contamination in the gas cell 20 can be considered to be low. Thus, the measurement of the sample signal value in step 406 is performed (S406). Step 406 and the subsequent steps are identical to step 200 and the subsequent steps of the first embodiment.

The prescribed value E1 can be set by determining the average of the relative transmitted light intensity of the reference gas represented on the vertical axis of FIG. 5B. The prescribed value E1 can be set to e.g. approximately 95% of this average.

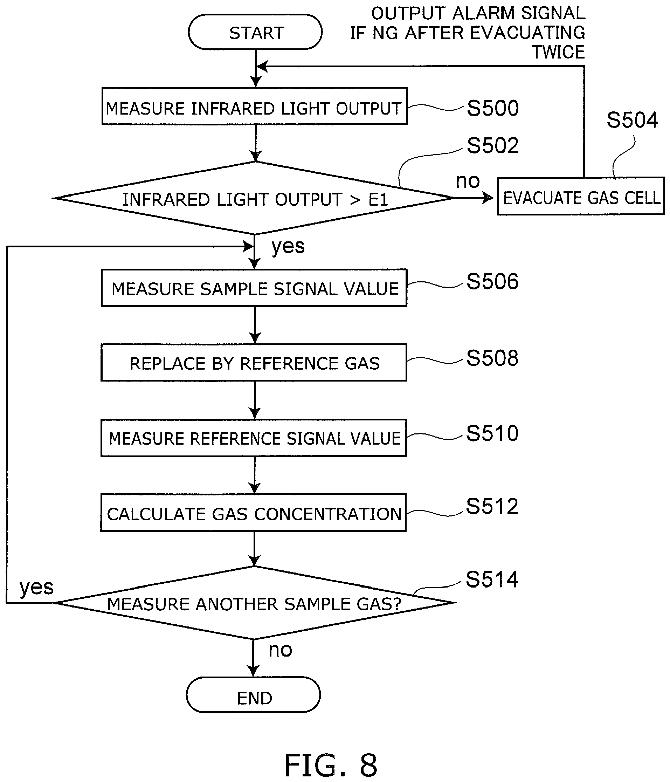

FIG. 8 is a flow chart of a gas analysis method according to a third embodiment.

The light intensity of infrared light may be less than or equal to the prescribed value E1 even after repeating evacuation and cleaning in the gas cell 20 a plurality of times (e.g., twice). Then, an alarm signal for e.g. requesting exchange of the adsorption column 54 can be outputted. Step 506 and the subsequent steps are identical to step 200 and the subsequent steps of the first embodiment. This dispenses with spending wasteful time for evacuation and cleaning.

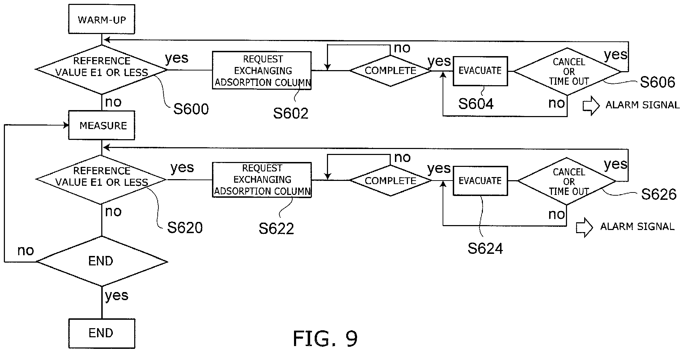

FIG. 9 is a flow chart showing an alternative example of the sequence for outputting an alarm signal.

For instance, the light intensity of infrared light may be less than or equal to the prescribed value E1 (S600). When it is less than or equal to the prescribed value E1 even after evacuating the gas cell a prescribed number of times, an alarm signal for requesting exchange of the adsorption column 54 is outputted (S622). Furthermore, evacuation is performed after exchanging the adsorption column 54 (S604). For instance, when the light intensity is less than or equal to the prescribed value E1 even after evacuation for a prescribed period of time, an alarm signal is further outputted.

The light intensity of infrared light may become higher than the prescribed value. Then, measurement of the sample gas SG and the reference gas RG is performed (S620). After the measurement, the gas cell 20 is filled with the reference gas RG. In this state, it is determined whether the light intensity is less than or equal to the prescribed value E1. Then, steps 622-626 can be performed as necessary.

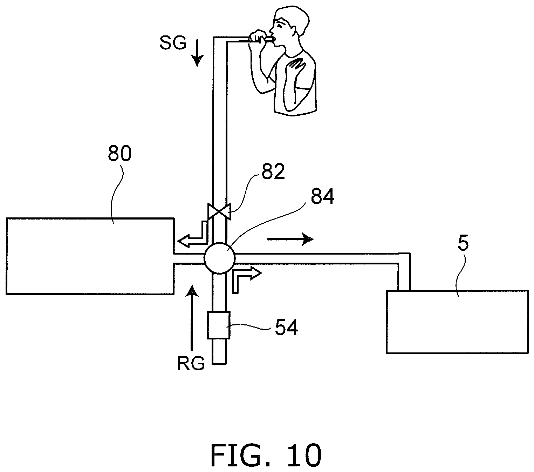

FIG. 10 is a configuration view of measuring the gas concentration off-line.

The sample gas SG is expired breath. By opening the valve 82, the sample gas SG is temporarily stored in the sampling bag 80. The capacity of the sampling bag can be set to e.g. 150 liters. The reference gas RG passes through the adsorption column 54 and is introduced into the gas analysis device 5 by the switching valve 84. Thus, gas analysis can be performed off-line.

The present embodiments provide a gas analysis method and a gas analysis device capable of measuring the gas concentration with high accuracy. For instance, measuring the concentration of a trace amount of gas contained in expired breath facilitates prevention and early detection of various diseases.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the invention.

**Claims**

1.  A gas analysis method comprising:

    irradiating a sample gas introduced into a gas cell with infrared light tuned to a wavelength corresponding to one absorption line of a target gas contained in the sample gas;
    measuring a sample signal value corresponding to intensity of transmitted light of the infrared light transmitted through the gas cell;

evacuating the sample gas in the gas cell and then replacing by a reference gas;
measuring a reference signal value corresponding to intensity of transmitted light of the infrared light transmitted through the reference gas; and
calculating gas concentration at the one absorption line from ratio of the sample signal value to the reference signal value.

2. The method according to claim 1, wherein
light intensity of the infrared light is measured before introducing the sample gas into the gas cell, and
when the intensity of the infrared light is less than or equal to a prescribed value, a control section outputs a signal for cleaning inside of the gas cell.

3. The method according to claim 2, wherein the reference gas is passed through an adsorption column and then introduced into the gas cell.

4. The method according to claim 3, wherein an alarm signal for exchanging the adsorption column is outputted when the intensity of the infrared light does not become higher than the prescribed value even after repeating cleaning the inside of the gas cell a prescribed number of times.

5. The method according to claim 1, wherein the infrared light is QCL laser light.

6. A gas analysis device comprising:

a light source configured to emit infrared light whose wavelength can be tuned to an absorption line of a target gas contained in a sample gas;
a gas cell to which either of a reference gas and the sample gas is selectively introduced, the gas cell including an incoming section irradiated with infrared light corresponding to one absorption line, and an outgoing section from which the infrared light travels out;
a detection section configured to detect a reference signal value corresponding to intensity of transmitted light of the reference gas and a sample signal value corresponding to intensity of transmitted light of the sample gas; and
a control section configured to select a gas introduction sequence so as to measure the sample signal value earlier than the reference signal value at the one absorption line, and to calculate gas concentration at the one absorption line from ratio of the sample signal value to the reference signal value.

7. The device according to claim 6, wherein when the detection section detects that the intensity of the transmitted light of the reference gas is less than or equal to a prescribed value, the control section outputs a signal for cleaning inside of the gas cell.

8. The device according to claim 7, further comprising:

an adsorption column configured to clean the reference gas.

9. The device according to claim 8, wherein an alarm signal for exchanging the adsorption column is outputted when the light intensity of the infrared light is less than or equal to the prescribed value even after repeating cleaning the inside of the gas cell a prescribed number of times.

10. The device according to claim 6, wherein the infrared light is QCL laser light.

## FIG. 1

START

MEASURE SAMPLE SIGNAL VALUE — S200

REPLACE BY REFERENCE GAS — S202

MEASURE REFERENCE SIGNAL VALUE — S204

CALCULATE GAS CONCENTRATION — S206

MEASURE ANOTHER SAMPLE GAS? — S208

yes

no

END

## FIG. 2

START

REPLACE GAS CELL REMAINING GAS BY REFERENCE GAS — S300

MEASURE REFERENCE SIGNAL VALUE — S302

INTRODUCE SAMPLE GAS — S304

MEASURE SAMPLE SIGNAL VALUE — S306

CALCULATE GAS CONCENTRATION — S308

S310
MEASURE ANOTHER SAMPLE GAS?

yes

no

END

FIG. 3

FIG. 4A

FIG. 4B

REFERENCE GAS TRANSMITTED LIGHT INTENSITY(a.u)

CUMULATIVE NUMBER OF TIMES

FIG. 5A

REFERENCE GAS TRANSMITTED LIGHT INTENSITY(a.u)

CUMULATIVE NUMBER OF TIMES

FIG. 5B

FIG. 6

START

MEASURE INFRARED LIGHT OUTPUT — S400

S402

INFRARED LIGHT OUTPUT > E1 — no → EVACUATE GAS CELL — S404

yes

MEASURE SAMPLE SIGNAL VALUE — S406

REPLACE BY REFERENCE GAS — S408

MEASURE REFERENCE SIGNAL VALUE — S410

CALCULATE GAS CONCENTRATION — S412

MEASURE ANOTHER SAMPLE GAS? — S414

yes

no

END

FIG. 7

START

OUTPUT ALARM SIGNAL
IF NG AFTER EVACUATING
TWICE

MEASURE INFRARED LIGHT OUTPUT — S500

S502

INFRARED LIGHT OUTPUT > E1 — no → EVACUATE GAS CELL — S504

yes

MEASURE SAMPLE SIGNAL VALUE — S506

REPLACE BY REFERENCE GAS — S508

MEASURE REFERENCE SIGNAL VALUE — S510

CALCULATE GAS CONCENTRATION — S512

S514
MEASURE ANOTHER SAMPLE GAS? — yes

no

END

FIG. 8

WARM-UP

REFERENCE
VALUE E1 OR LESS —— yes —— REQUEST
EXCHANGING
ADSORPTION COLUMN —— COMPLETE —— no

REFERENCE
VALUE E1 OR LESS —— no

S600

REQUEST
EXCHANGING
ADSORPTION COLUMN

S602

COMPLETE —— yes —— EVACUATE —— CANCEL
OR
TIME OUT —— yes

S604

no —— ⇨ ALARM SIGNAL

S606

MEASURE

REFERENCE
VALUE E1 OR LESS —— yes —— REQUEST
EXCHANGING
ADSORPTION COLUMN —— no —— COMPLETE —— yes —— EVACUATE —— CANCEL
OR
TIME OUT —— yes

no

S620

S622

S624

no —— ⇨ ALARM SIGNAL

S626

END —— no

yes

END

FIG. 9

FIG. 10

## INTERNATIONAL SEARCH REPORT

| International application No. |
| --- |
| PCT/JP2015/076995 |

**A. CLASSIFICATION OF SUBJECT MATTER**
*G01N21/3504*(2014.01)i

According to International Patent Classification (IPC) or to both national classification and IPC

**B. FIELDS SEARCHED**

Minimum documentation searched (classification system followed by classification symbols)
G01N21/3504

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP 2004-177323 A (Shimadzu Corp.),<br>24 June 2004 (24.06.2004),<br>paragraphs [0005], [0025] to [0027]; fig. 1, 2,<br>5, 6<br>(Family: none) | 1,3,5,6,8,10<br>2,4,7,9 |
| Y | JP 2011-191164 A (Fuji Electric Co., Ltd.),<br>29 September 2011 (29.09.2011),<br>paragraph [0051]<br>(Family: none) | 2,4,7,9 |
| Y | JP 63-167254 A (The Institute of Physical and<br>Chemical Research),<br>11 July 1988 (11.07.1988),<br>page 4, lower left column, lines 2 to 6<br>(Family: none) | 2,4,7,9 |

☐ Further documents are listed in the continuation of Box C. ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>02 December 2015 (02.12.15) | Date of mailing of the international search report<br>15 December 2015 (15.12.15) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4108297 B